Europäisches Patentamt

European Patent Office

Office européen des brevets

(11) Publication number: **0 584 421 A1**

## EUROPEAN PATENT APPLICATION

(21) Application number: **92402326.0**

(22) Date of filing: **21.08.92**

(51) Int. Cl.5: **C12N 15/13**, C07K 15/06, C12P 21/08, C12N 5/10, A61K 39/395

(43) Date of publication of application:
**02.03.94 Bulletin 94/09**

(84) Designated Contracting States:
**AT BE CH DE DK ES FR GB GR IE IT LI LU MC NL PT SE**

(71) Applicant: **Casterman, Cécile**
**Vijversweg 15**
**B-1640 Sint-Genesius-Rode(BE)**
Applicant: **HAMERS, Raymond**
**Vijversweg 15**
**B-1640 Sint-Genesius-Rode(BE)**

(72) Inventor: **Casterman, Cécile**
**Vijversweg 15**
**B-1640 Sint-Genesius-Rode(BE)**
Inventor: **HAMERS, Raymond**
**Vijversweg 15**
**B-1640 Sint-Genesius-Rode(BE)**

(74) Representative: **Desaix, Anne et al**
**Ernest Gutmann - Yves Plasseraud S.A.**
**3, rue Chauveau-Lagarde**
**F-75008 Paris (FR)**

(54) **Immunoglobulins devoid of light chains.**

(57) The invention relates to isolated immunoglobulin, characterized in that it comprises two heavy polypeptide chains sufficient for the formation of a complete antigen binding site or several antigen binding sites, this immunoglobulin being further devoid of light polypeptide chains.

The invention relates to new isolated immunoglobulins which are devoid of light polypeptide chains. These immunoglobulins do not consist of the degradation product of immunoglobulins composed of both heavy polypeptide and light polypeptide chains but to the contrary, the invention defines a new member of the family of the immunoglobulins, especially a new type of molecules capable of being involved in the immune recognition. Such immunoglobulins can be used for several purposes, especially for diagnosis or therapeutical purposes including protection against pathological agents or regulation of the expression or activity of proteins.

Up to now the structure proposed for immunoglobulins consists of a four-chain model referring to the presence of two identical light polypeptide chains (light chains) and two identical heavy polypeptide chains (heavy chains) linked together by disulfide bonds to form a y- or T-shaped macromolecules. These chains are composed of a constant region and a variable region, the constant region being subdivided in several domains. The two heavy polypeptide chains are usually linked by disulphide bounds in a so-called "hinge region" situated between the first and second domains of the constant region.

Among the proteins forming the class of the immunoglobulins, most of them are antibodies and accordingly present an antigen binding site

According to the four-chain model, the antigen binding site of an antibody is located in the variable domains of each heavy and light chains, and requires the association of the heavy and the light chains variable domains.

For the definition of these four-chain model immunoglobulins, reference is made to Roitt. I et al (Immunology-second-Edition Gower Medical Publishing USA, 1989). Reference is especially made to the part concerning the definition of the four-chain immunoglobulins, their polypeptidic and genetic structures, the definition of their variable and constant regions and the obtention of the fragments produced by enzymatic degradation according to well known techniques.

The inventors have surprisingly established that different molecules can be isolated from animals which naturally produce them, which molecules have functional properties of immunoglobulins these functions being in some cases related to structural elements which are distinct from those involved in the function of four-chain immunoglobulins due for instance to the absence of light chains.

The invention relates to two-chain model immunoglobulins which neither correspond to fragments obtained for instance by the degradation in particular the enzymatic degradation of a natural four-chain model immunoglobulin, nor correspond to the expression in host cells, of DNA coding for the constant or the variable region of a natural four-chain model immunoglobulin or a part of these regions, nor correspond to antibodies produced in lymphopaties for example in mice, rats or human.

E.S. Ward et al (1) have described some experiments performed on variable domains of heavy polypeptide chains ($V_H$) or/and light polypeptide chains ($V_K/F_V$) to test the ability of these variable domains, to bind specific antigens. For this purpose, a library of $V_H$ genes was prepared from the spleen genomic DNA of mice previously immunized with these specific antigens.

Ward et al have described in their publication that $V_H$ domains are relatively sticky, presumably due to the exposed hydrophobic surface normally capped by the $V_K$ or $V_\lambda$ domains. They consequently envisage that it should be possible to design $V_H$ domains having improved properties and further that $V_H$ domains with binding activities could serve as the building blocks for making variable fragments (Fv fragments) or complete antibodies.

The invention does not start from the idea that the different fragments (light and heavy chains) and the different domains of these fragments of four-chain model immunoglobulin can be modified to define new or improved antigen binding sites or a four-chain model immunoglobulin.

The inventors have determined that immunoglobulins can have a different structure than the known four-chain model and that such different immunoglobulins offer new means for the preparation of diagnosis reagents, therapeutical agents or any other reagent for use in research or industrial purposes.

Thus the invention provides new immunoglobulins which are capable of showing functional properties of four-chain model immunoglobulins although their structure appears to be more appropriate in many circumstances for their use, their preparation and in some cases for their modification. Moreover these molecules can be considered as lead structures for the modification of other immunoglobulins. The advantages which are provided by these immunoglobulins comprise the possibility to prepare them with an increased facility.

The invention accordingly relates to isolated immunoglobulins characterized in that they comprise two heavy polypeptide chains sufficient for the formation of a complete antigen binding site or several antigen binding sites, these immunoglobulins being further devoid of light polypeptide chains. By "a complete antigen binding site" it is meant according to the invention, a site which will alone allow the recognition and complete binding of an antigen. This could be verified by any known method regarding the testing of the binding affinity.

These immunoglobulins which can be isolated from animals, will be sometimes called "heavy-chain immunoglobulins" in the following pages. In a preferred embodiment of the invention, these immunoglobulins are in a pure form.

According to a preferred embodiment, the immunoglobulins are characterized in that their variable regions contain in position 45, an amino-acid which is different from leucine, proline or glutamine residue.

Moreover as isolated and purified products the heavy-chain immunoglobulins are not obtained from lymphocytes of animals nor from lymphocytes of a human patient suffering from lymphopathies. Such immunoglobulins produced in lymphopathies have apparently no antigen binding site.

The two heavy polypeptide chains of these immunoglobulins can be linked by a hinge region according to the definition of Roitt et al.

In a particular embodiment of the invention, immunoglobulins corresponding to the above-defined molecules are capable of acting as antibodies.

The antigen binding site(s) of the immunoglobulins of the invention are located in the variable region of the heavy chain.

In a particular group of these immunoglobulins each heavy polypeptide chain contains one antigen binding site on its variable region, and these sites correspond to the same amino-acid sequence.

In a further embodiment of the invention the immunoglobulins are characterized in that their heavy polypeptide chains contain a variable region ($V_H$) and a constant region ($C_H$) according to the definition of Roitt et al, but are devoid of the first domain of their constant region. This first domain of the constant region is called $C_H1$.

These immunoglobulins having no $C_H1$ domain are such that the variable region of their chains is directly linked to the hinge region at the C-terminal part of the variable region.

The immunoglobulins of the type described hereabove can comprise type G immunoglobulins and especially immunoglobulins which are defined as immunoglobulins of class 2 (IgG2) or immunoglobulins of class 3 (IgG3).

The absence of the light chain and of the first constant domain lead to a modification of the nomenclature of the immunoglobulin fragments obtained by enzymatic digestion, according to Roitt et al.

The terms Fc and pFc on the one hand, Fc' and pFc' on the other hand corresponding respectively to the papain and pepsin digestion fragments are maintained.

The terms Fab F(ab)₂ F(ab')₂ Fabc, Fd and Fv are no longer applicable in their original sense as these fragments have either a light chain, the variable part of the light chain or the $C_H1$ domain.

The fragments obtained by papain digestion and composed of the $V_H$ domain and the hinge region will be called $FV_Hh$ or $F(V_Hh)_2$ depending upon whether or not they remain linked by the disulphide bonds.

Interestingly immunoglobulins of the invention can be originating from animals of the camelid family. The inventors have found out that the heavy-chain immunoglobulins which are present in camelids are not associated with a pathological situation which would induce the production of abnormal antibodies with respect to the four-chain immunoglobulins. On the basis of a comparative study of old world camelids (Camelus bactrianus and Camelus dromaderius) and new world camelids (for example Lama Paccos, Lama Glama, and Lama Vicugna) the inventors have shown that the immunoglobulins of the invention, which are devoid of light polypeptide chains are found in all species. Nevertheless differences may be apparent in molecular weight of these immunoglobulins depending on the animals. Especially the molecular weight of a heavy chain contained in these immunoglobulins can be from approximately 43 kd to approximately 47 kd, in particular 45 kd.

Advantageously the heavy-chain immunoglobulins of the invention are secreted in blood of camelids.

Immunoglobulins according to the invention are obtainable by purification from serum of camelids and a process for the purification is described in details in the examples. In the case where the immunoglobulins are obtained from Camelids, the invention relates to immunoglobulins which are not in their natural biological environment.

According to the invention immunoglobulin IgG2 as obtainable by purification from the serum of camelids can be characterized in that :
- it is not adsorbed by chromatography on Protein G Sepharose column,
- it is adsorbed by chromatography on Protein A Sepharose column,
- it has a molecular weight of around 100 Kd after elution with a pH 4.5 buffer (0.15 M NaCl, 0.58% acetic acid adjusted to pH 4.5 by NaOH),
- it consists of heavy γ2 polypeptide chains of a molecular weight of around 46 kd preferably 45 after reduction.

According to a further embodiment of the invention another group of immunoglobulins corresponding to IgG3, as obtainable by purification from the serum of Camelids is characterized in that the immunoglobulin :
- is adsorbed by chromatography on a Protein A Sepharose column,
- has a molecular weight of around 100 Kd after elution with a 3.5 buffer (0.15 M NaCl, 0.58% acetic acid),
- is adsorbed by chromatography on a Protein G Sepharose column and eluted with pH 3.5 buffer (0.15 M NaCl, 0.58% acetic acid).
- consists of heavy $\gamma$3 polypeptide chains of a molecular weight of around 45 Kd in particular between 43 and 47 kd after reduction.

The immunoglobulins of the invention which are devoid of light chains, nevertheless comprise on their heavy chains a constant region and a variable region. The constant region comprises different domains.

The variable region of immunoglobulins of the invention comprises frameworks (FW) and complementarity determining regions (CDR), especially 4 frameworks and 3 complementarity regions. It distinguishes from the four-chain immunoglobulins especially by the fact that this variable region can itself contain an antigen binding site or several, without contribution of the variable region of a light chain which is absent.

The amino-acid sequences of frameworks 1 and 4 comprise among others respectively amino-acid sequences which can be selected from the following : for the framework 1 domain

G G S V Q T G G S L R L S C E I S G L T F D

G G S V Q T G G S L R L S C A V S G F S F S

G G S E Q G G G S L R L S C A I S G Y T Y G

G G S V Q P G G S L T L S C T V S G A T Y S

G G S V Q A G G S L R L S C T G S G F P Y S

G G S V Q A G G S L R L S C V A G F G T S

G G S V Q A G G S L R L S C V S F S P S S

for the framework 4 domain

W G Q G T Q V T V S S

W G Q G T L V T V S S

W G Q G A Q V T V S S

W G Q G T Q V T A S S

R G Q G T Q V T V S L

As stated above, the immunoglobulins of the invention are preferably devoid of the totality of their $C_H1$ domain.

Such immunoglobulins comprise $C_H2$ and $C_H3$ domains in the C-terminal region with respect to the hinge region.

According to a particular embodiment of the invention the constant region of the immunoglobulins comprises $C_H2$ and $C_H3$ domains comprising an amino-acid sequence selected from the following : for the $C_H2$ domain:

APELLGGPTVFIFPPKPKDVLSITLTP

APELPGGPSVFVFPTKPKDVLSISGRP

APELPGGPSVFVFPPKPKDVLSISGRP

APELLGGPSVFIFPPKPKDVLSISGRP

for the $C_H3$ domain:

GQTREPQVYTLA

Interestingly the inventors have shown that the hinge region of the immunoglobulins of the invention can present variable lengths. When these immunoglobulins act as antibodies, the length of the hinge region will participate to the determination of the distance separating the antigen binding sites.

Preferably an immunoglobulin according to the invention is characterized in that its hinge region comprises from 0 to 50 amino-acids.

Particular sequences of hinge region of the immunoglobulins of the invention are the following.

GTNEVCKCPKCP

or,

EPKIPQPQPKPQPQPQPQPKPQPKPEPECTCPKCP

The short hinge region corresponds to an IgG3 molecule and the long hinge sequence corresponds to an IgG2 molecule.

Isolated $V_H$ derived from heavy chain immunoglobulins or $V_H$ libraries corresponding to the heavy chain immunoglobulins can be distinguished from $V_H$ cloning of four-chain model immunoglobulins on the basis of sequence features characterizing heavy chain immunoglobulins.

The camel heavy - chain immunoglobulin $V_H$ region shows a number of differences with the $V_H$ regions derived from 4-chain immunoglobulins from all species examined. At the levels of the residues involved in the $V_H/V_L$ interactions, an important difference is noted at the level of position 45 (FW) which is practically always leucine in the 4-chain immunoglobulins (98%), the other amino acids at this position being proline (1%) or glutamine (1%).

In the camel heavy-chain immunoglobulin, in the sequences examined at present, leucine at position 45 is only found once. In the other cases, it is replaced by arginine, cysteine or glutamic acid residue. The presence of charged amino acids at this position should contribute to making the $V_H$ more soluble.

The replacement by camelid specific residues such as those of position 45 appears to be interesting for the construction of engineered $V_H$ regions derived from the $V_H$ repertoire of 4-chain immunoglobulins.

A second feature specific of the camelid $V_H$ domain is the frequent presence of a cysteine in the $CDR_3$ region associated with a cysteine in the $CDR_1$ position 31 or 33 or $FW_2$ region at position 45. The possibility of establishing a disulphide bond between the $CDR_3$ region and the rest of the variable domain would contribute to the stability and positioning of the binding site.

With the exception of a single pathogenic myeloma protein (DAW) such a disulphide bond has never been encountered in immunoglobulin V regions derived from 4 chain immunoglobulins.

The heavy-chain immunoglobulins of the invention have further the particular advantage of being not sticky. Accordingly these immunoglobulins being present in the serum, aggregate much less than isolated heavy chains of a four-chain immunoglobulins. The immunoglobulins of the invention are soluble to a concentration above 0.5 mg/ml, preferably above 1 mg/ml and more advantageously above 2 mg/ml.

These immunoglobulins further bear an extensive antigen binding repertoire and undergo affinity and specificity maturation in vivo. Accordingly they allow the isolation and the preparation of antibodies having defined specificity, regarding determined antigens.

Another interesting property of the immunoglobulins of the invention is that they can be modified and especially humanized. Especially it is possible to replace all or part of the constant region of these immunoglobulins by all or part of a constant region of a human antibody. For example the $C_H2$ and/or $C_H3$ domains of the immunoglobulin could be replaced by the $C_H2$ and/or $C_H3$ domains of the IgG $\gamma3$ human immunoglobulin.

In such humanized antibodies it is also possible to replace a part of the variable sequence, namely one or more of the framework residues which do not intervene in the binding site by human framework residues, or by a part of a human antibody.

Conversely features (especially peptide fragments) of heavy-chain immunoglobulin $V_H$ regions, could be introduced into the $V_H$ or $V_L$ regions derived from four-chain immunoglobulins with for instance the aim of achieving greater solubility of the immunoglobulins.

The invention further relates to a fragment of an immunoglobulin which has been described hereabove and especially to a fragment selected from the following group :
- a fragment corresponding to one heavy polypeptide chain of an immunoglobulin devoid of light chains,
- fragments obtained by enzymatic digestion of the immunoglobulins of the invention, especially those obtained by partial digestion with papaïn leading to the Fc fragment (constant fragment) and leading to $Fv_Hh$ fragment (containing the antigen binding sites of the heavy chains) or its dimer $F(V_Hh)_2$, or a fragment obtained by further digestion with papaïn of the Fc fragment, leading to the pFc fragment corresponding to the C-terminal part of the Fc fragment,
- homologous fragments obtained with other proteolytic enzymes,
- a fragment of at least 10 preferably 20 amino acids of the variable region of the immunoglobulin, or the complete variable region, especially a fragment corresponding to the isolated $V_H$ domains or to the $V_H$ dimers linked to the hinge disulphide,
- a fragment corresponding to the hinge region of the immunoglobulin,or to at least 6 amino acids of this hinge region,
- a fragment of the hinge region comprising a repeated sequence of Pro-X,
- a fragment corresponding to at least 10 preferably 20 amino acids of the constant region or to the complete constant region of the immunoglobulin.

The invention also relates to a fragment comprising a repeated sequence, Pro-X which repeated sequence contains at least 3 repeats of Pro-X, X being any amino-acid and preferably Gln (glutamine), Lys (lysine) or Glu (acide glutamique); a particular repeated fragment is composed of a 12-fold repeat of the sequence Pro-X.

Such a fragment can be advantageously used as a link between different types of molecules.

The amino-acids of the Pro-X sequence are chosen among any natural or non natural amino-acids.

The fragments can be obtained by enzymatic degradation of the immunoglobulins. They can also be obtained by expression in cells or organisms, of nucleotide sequence coding for the immunoglobulins, or they can be chemically synthetized.

The invention also relates to anti-idiotypes antibodies belonging to the heavy chain immunoglobulin classes. Such anti-idiotypes can be produced against human or animal idiotypes. A property of these anti-idiotypes is that they can be used as idiotypic vaccines, in particular for vaccination against glycoproteins or glycolipids and where the carbohydrate determines the epitope.

The invention also relates to anti-idiotypes capable of recognizing idiotypes of heavy-chain immunoglobulins.

Such anti-idiotype antibodies can be either syngeneic antibodies or allogenic or xenogeneic antibodies.

The invention also concerns nucleotide sequences coding for all or part of a protein which amino-acid sequence comprises a peptide sequence selected from the following :

```
G  G  S  V  Q  T  G  G  S  L  R  L  S  C  E  I  S  G  L  T  F  D
G  G  S  V  Q  T  G  G  S  L  R  L  S  C  A  V  S  G  F  S  F  S
G  G  S  E  Q  G  G  G  S  L  R  L  S  C  A  I  S  G  Y  T  Y  G
G  G  S  V  Q  P  G  G  S  L  T  L  S  C  T  V  S  G  A  T  Y  S
G  G  S  V  Q  A  G  G  S  L  R  L  S  C  T  G  S  G  F  P  Y  S
G  G  S  V  Q  A  G  G  S  L  R  L  S  C  V  A  G  F  G  T  S
G  G  S  V  Q  A  G  G  S  L  R  L  S  C  V  S  F  S  P  S  S


W  G  Q  G  T  Q  V  T  V  S  S
W  G  Q  G  T  L  V  T  V  S  S
W  G  Q  G  A  Q  V  T  V  S  S
W  G  Q  G  T  Q  V  T  A  S  S
R  G  Q  G  T  Q  V  T  V  S  L


APELLGGPSVFVFPPKPKDVLSISGXPK
APELPGGPSVFVFPTKPKDVLSISGRPK
APELPGGPSVFVFPPKPKDVLSISGRPK
APELLGGPSVFIFPPKPKDVLSISGRPK


GQTREPQVYTLAPXRLEL
GQPREPQVYTLPPSRDEL
GQPREPQVYTLPPSREEM
GQPREPQVYTLPPSQEEM


                    VTVSSGTNEVCKCPKCPAPELPGGPSVFVFP
or,
        VTVSSEPKIPQPQPKPQPQPQPQPKPQPKPEPECTCPKCPAPELLGGPSVFIFP


        GTNEVCKCPKCP
        APELPGGPSVFVFP
        EPKIPQPQPKPQPQPQPQPKPQPKPEPECTCPKCP
        APELLGGPSVFIFP
```

Such nucleotide sequences can be deduced from the amino-acid sequences taking into account the deneneracy of the genetic code. They can be synthetized or isolated from cells producing immunoglobulins of the invention.

A procedure for the obtention of such DNA sequences is described in the examples.

The invention also contemplates RNA, especially mRNA sequences corresponding to these DNA sequences, and also corresponding cDNA sequences.

The nucleotide sequences of the invention can further be used for the preparation of primers appropriate for the detection in cells or screening of DNA or cDNA libraries to isolate nucleotide sequences coding for immunoglobulins of the invention.

Such nucleotide sequences can be used for the preparation of recombinant vectors and the expression of these sequences contained in the vectors by host cells especially prokaryotic cells like bacteria or also eukaryotic cells and for example CHO cells, insect cells, simian cells like Vero cells, or any other mammalian cells. Especially the fact that the immunoglobulins of the invention are devoid of light chains permits to secrete them in eukaryotic cells since there is no need to have recourse to the step consisting in the formation of the BIP protein which is required in the four-chain immunoglobulins.

The inequacies of the known methods for producing monoclonal antibodies or immunoglobulins by recombinant DNA technology comes from the necessity in the vast majority of cases to clone simultaneously the $V_H$ and $V_L$ domains corresponding to the specific binding site of 4 chain immunoglobulins. The animals and especially camelids which produce heavy-chain immunoglobulins according to the invention, and possibly other vertebrate species are capable of producing heavy-chain immunoglobulins of which the binding site is located exclusively in the $V_H$ domain. Unlike the few heavy-chain immunoglobulins produced in other species by chain separation or by direct cloning, the camelid heavy-chain immunoglobulins have undergone extensive maturation in vivo. Moreover their V region has naturally evolved to function in absence of the $V_L$. They are therefore ideal for producing monoclonal antibodies by recombinant DNA technology. As the obtention of specific antigen binding clones does not depend on a stochastic process necessitating a very large number of recombinant cells, this allows also a much more extensive examination of the repertoire.

This can be done at the level of the non rearranged $V_H$ repertoire using DNA derived from an arbitrarily chosen tissue or cell type or at the level of the rearranged $V_H$ repertoire, using DNA obtained from B lymphocytes. More interesting however is to transcribe the mRNA from antibody producing cells and to clone the cDNA with or without prior amplification into an adequate vector. This will result in the obtention of antibodies which have already undergone affinity maturation.

The examination of a large repertoire should prove to be particularly useful in the search for antibodies with catalytic activities.

The invention thus provides libraries which can be generated in a way which includes part of the hinge sequence, the identification is simple as the hinge is directly attached to the $V_H$ domain.

These libraries can be obtained by cloning cDNA from lymphoid cells with or without prior PCR amplification. The PCR primers are located in the promoter, leader or framework sequences of the $V_H$ for the 5' primer and in the hinge, $CH_2$, $CH_3$, 3' untranslated region or polyA tail for the 3' primer. A size selection of amplified material allows the construction of a library limited to heavy chain immunoglobulins.

In a particular example, the following 3' primer in which a KpnI site has been constructed and which corresponds to amino-acids 313 to 319 (CGC CAT CAA GGT AAC AGT TGA) is used in conjunction with mouse $V_H$ primers described by Sestry et al and containing a Xho site

AG GTC CAG CTG CTC GAG TCT GG

AG CTC CAG CTG CTC GAG TCT GG

AG GTC CAG CTT CTC GAG TCT GG

XhoI site

These primers yield a library of camelid heavy chain immunoglobulins comprising the $V_H$ region (related to mouse or human subgroup III), the hinge and a section of $CH_2$.

In another example, the cDNA is polyadenylated at its 5' end and the mouse specific $V_H$ primers are replaced by a poly T primer with an inbuilt XhoI site, at the level of nucleotide 12.

$CTCGAGT_{12}$.

The same 3' primer with a KpnI site is used.

This method generates a library containing all subgroups of immunoglobulins.

Part of the interest in cloning a region encompassing the hinge-$CH_2$ link is that in both γ2 and γ3, a Sac site is present immediately after the hinge. This site allows the grafting of the sequence coding for the $V_H$ and the hinge onto the Fc region of other immunoglobulins, in particular the human $IgG_1$ and $IgG_3$ which have the same amino acid sequence at this site ($Glu_{246}$ $Leu_{247}$).

As an example, the invention contemplates a cDNA library composed of nucleotide sequences coding for a heavy-chain immunoglobulin , such as obtained by performing the following steps:

a) treating a sample containing lymphoïd cells, especially periferal, lymphocytes, spleen cells, lymph nodes or another lyphoïd tissue from a healthy animal, especially selected among the Camelids, in order to separated the lymphoid cells,

b) separating polyadenylated RNA from the other nucleic acids and components of the cells,

c) reacting the obtained RNA with a reverse transcriptase in order to obtain the corresponding cDNA,

d) contacting the amplified DNA of step e) with 5' primers corresponding to mouse $V_H$ domain of four-chain immunoglobulins, which primer contains a determined restriction site, for example an XhoI site and with 3' primers corresponding to the N-terminal part of a $C_H2$ domain containing a KpnI site,

e) amplifying the DNA,

f) cloning the amplified sequence in a vector, especially in a bluescript vector,

g) recovering the clones hybridizing with a probe corresponding to the sequence coding for a constant domain from an isolated heavy-chain immunoglobulin.

This cloning gives rise to clones containing DNA sequences including the sequence coding for the hinge. It thus permits the characterization of the subclass of the immunoglobulin and the SacI site useful for grafting the $FV_Hh$ to the Fc region.

The recovery of the sequences coding for the heavy-chain immunoglobulins can also be achieved by the selection of clones containing DNA sequences having a size compatible with the lack of the $C_H1$ domain.

It is possible according to another embodiment of the invention, to add the following steps between steps c) and d) of the above process:

- in the presence of a DNA polymerase and of deoxyribonucleotide triphosphates, contacting said cDNA with oligonucleotide degenerated primers, which sequences are capable of coding for the hinge region and N-terminal $V_H$ domain of an immunoglobulin, the primers being capable of hybridizing with the cDNA and capable of initiating the extension of a DNA sequence complementary to the cDNA used as template,
- recovering the amplified DNA.

The invention also relates to a DNA library composed of nucleotide sequences coding for a heavy-chain immunoglobulin, such as obtained from cells with rearranged immunoglobulin genes.

In a preferred embodiment of the invention, the library is prepared from cells from an animal previously immunized against a determined antigen. This allows the selection of antibodies having a preselected specificity for the antigen used for immunization.

In another embodiment of the invention, the amplification of the cDNA is not performed prior to the cloning of the cDNA.

The heavy chain of the four-chain immunoglobulins remains sequestered in the cell by a chaperon protein (BIP) until it has combined with a light chain. The binding site for the chaperon protein is the $C_H1$ domain. As this domain is absent from the heavy chain immunoglobulins, their secretion is independent of the presence of the BIP protein or of the light chain. Moreover the inventors have shown that the obtained immunoglobulins are not sticky and accordingly will not abnormally aggregate.

The invention also relates to a process for the preparation of a monoclonal antibody directed against a determined antigen, the antigen binding site of the antibody consisting of heavy polypeptide chains and which antibody is further devoid of light polypeptide chains, which process comprises :

- immortalizing lymphocytes, obtained for example from the peripheral blood of Camelids previously immunized with a determined antigen, with myeloma cells, in order to form a hybridoma,
- culturing the immortalized cells (hybridoma) formed and recovering the cells producing the antibodies having the desired specificity.

The preparation of antibodies can also be performed without a previous immunization of Camelids.

^LPage 21

According to another process for the preparation of antibodies, the recourse to the technique of the hybridoma cell is not required.

According to such process, antibodies are prepared in vitro and they can be obtained by a process comprising the steps of :

- cloning into vectors, especially into phages and more particularly filamentous bacteriophages, DNA or cDNA sequences obtained from lymphocytes especially PBLs of Camelids previously immunized with determined antigens,
- transforming prokaryotic cells with the above vectors in conditions allowing the production of the antibodies,
- selecting the antibodies for their heavy-chain structure and further by subjecting them to antigen-affinity selection,
- recovering the antibodies having the desired specificity,

In another embodiment of the invention the cloning is performed in vectors, especially into plasmids coding for bacterial membrane proteins. Procaryotic cells are then transformed with the above vectors in conditions allowing the expression of antibodies in their membrane.

9

The positive cells are further selected by antigen affinity selection.

In yet another embodiment of the invention, the cloning vector is a plasmid or a eukaryotic virus vector and the cells to be transformed are eukaryotic cells, especially yeast cells, mammalian cells for example CHO cells or simian cells such as Vero cells, insect cells, plant cells, or protozoan cells.

For more details concerning the procedure to be applied in such a case, reference is made to the publication of Marks et al, J. Mol. Biol. 1991, 222:581-597.

Furthermore, starting from the immunoglobulins of the invention, or from fragments thereof, new immunoglobulins or derivatives can be prepared.

Accordingly immunoglobulins replying to the above given definitions can be prepared against determined antigens. Especially the invention provides monoclonal antibodies or polyclonal antiserums devoid of light polypeptide chains and directed against determined antigens and for example against antigens of pathological agents such as bacteria, viruses or parasites. As example of antigens or antigenic determinants against which antibodies could be prepared, one can cite the envelope glycoproteins of viruses or peptides thereof, such as the external envelope glycoprotein of a HIV virus, the surface antigen of the hepatitis B virus.

Immunoglobulins of the invention can also be directed against a protein, hapten, carbohydrate or nucleic acid.

Particular antibodies according to the invention are directed against the galactosyl$\alpha$-1-3-galactose epitope.

The immunoglobulins of the invention allow further the preparation of combined products such as the combination of the heavy-chain immunoglobulin or a fragment thereof with a toxin, an enzyme, a drug, a hormone.

As example one can prepare the combination of a heavy-chain immunoglobulin bearing an antigen binding site recognizing a myeloma immunoglobulin epitope with the abrin or mistletoe lectin toxin. Such a construct would have its uses in patient specific therapy.

Another advantageous combination is that one can prepare between a heavy-chain immunoglobulins recognizing an insect gut antigen with a toxin specific for insects such as the toxins of the different serotypes of Bacillus thuringiensis or Bacillus sphaericus. Such a construct cloned into plants can be used to increase the specificity or the host range of existing bacterial toxins.

The invention also proposes antibodies having different specificities on each heavy polypeptide chains. These multifunctional, especially bifunctional antibodies could be prepared by combining two heavy chains of immunoglobulins of the invention or one heavy chain of an immunoglobulin of the invention with a fragment of a four-chain model immunoglobulin.

The invention also provides hetero-specific antibodies which can be used for the targetting of drugs or any biological substance like hormones. In particular they can be used to selectively target hormones or cytokines to a limited category of cells. Examples are a combination of a murine or human antibody raised against interleukin 2 ($IL_2$) and a heavy-chain antibody raised against $CD_4$ cells. This could be used to reactivate $CD_4$ cells which have lost their $IL_2$ receptor.

In a particular embodiment of the invention, the hinge region of IgG2 immunoglobulins according to the invention is semi-rigid and is thus appropriate for coupling proteins. In such an application proteins or peptides can be linked to various substances, especially to ligands through the hinge region used as spacer. Advantageously the fragment comprises at least 6 amino acids.

According to the invention it is interesting to use a sequence comprising a repeated sequence Pro-X, X being any amino-acid and preferably Gln, Lys or Glu, especially a fragment composed of at least a 3-fold repeat and preferably of a 12-fold repeat, for coupling proteins to ligand, or for assembling different protein domains.

The hinge region or a fragment thereof can also be used for coupling proteins to ligands or for assembling different protein domains.

Usual techniques for the coupling are appropriate and especially reference may be made to the technique of protein engineering by assembling cloned sequences.

The antibodies according to this invention could be used as reagents for the diagnosis in vitro or by imaging techniques. The immunoglobulins of the invention could be labelled with radio-isotopes, chemical or enzymatic markers or chemiluminescent markers.

As example and especially in the case of detection or observation with the immunoglobulins by imaging techniques, a label like technetium, especially technitium 99 is advantageous. This label can be used for direct labelling by a coupling procedure with the immunoglobulins or fragments thereof or for indirect labelling after a step of preparation of a complex with the technitium.

Other interesting radioactive labels are for instance indium and especially indium 111, or iodine, especially $I^{131}$, $I^{125}$ and $I^{123}$.

For the description of these techniques reference is made to the FR patent application published under number 2649488.

The invention also concerns monoclonal antibodies reacting with anti-idiotypes of the above-described antibodies.

The invention also concerns cells or organisms in which heavy-chain immunoglobulins have been cloned. Such cells or organisms can be used for the purpose of producing heavy-chain immunoglobulins having a desired preselected specificity, or corresponding to a particular repertoire. They can also be produced for the purpose of modifying the metabolism of the cell which expresses them. In the case of modification of the metabolism of cells transformed with the sequences coding for heavy-chain immunoglobulins, these produced heavy-chain immunoglobulins are used like antisense DNA. Antisense DNA is usually involved in blocking the expression of certain genes such as for instance the variable surface antigen of trypanosomes or other pathogens. Likewise, the production or the activity of certain proteins or enzymes could be inhibited by expressing antibodies against this protein or enzyme within the same cell.

The invention also relates to a modified 4-chain immunoglobulin or fragments thereof in more specially the $V_H$ domain, the $V_H$ regions of which has been partialy replaced by specific sequences or amino acids of heavy chain immunoglobulins.

A modified $V_H$ domain of a four-chain immunoglobulin, is characterized in that the leucine, proline or glutamine in position 45 of the $V_H$ regions has been replaced by other amino acids and preferably by arginine, glutamic acid or cysteine.

A further modified $V_H$ or $V_L$ domain of a four-chain immunoglobulin, is characterized by linking of CDR loops together or to FW regions by the introduction of paired cysteines, the CDR region being selected between the $CDR_1$ and the $CDR_3$, the FW region being the $FW_2$ region, and especially in which one of the cysteines introduced is in position 31, 33 of $FR_2$ or 45 of $CDR_2$ and the other in $CDR_3$.

Especially the introduction of paired cysteines is such that the $CDR_3$ loop is linked to the FW2 or CDR1 domain and more especially the cysteine of the CDR3 of the $V_H$ is linked to a cysteine in position 31 or 33 of FW2 or in position 45 of CDR2.

In another embodiment of the invention, plant cells can be modified by the heavy-chain immunoglobulins according to the invention, in order that they acquire new properties or increased properties.

Other advantages and characteristics of the invention will become apparent in the examples and figures which follow.

## FIGURES

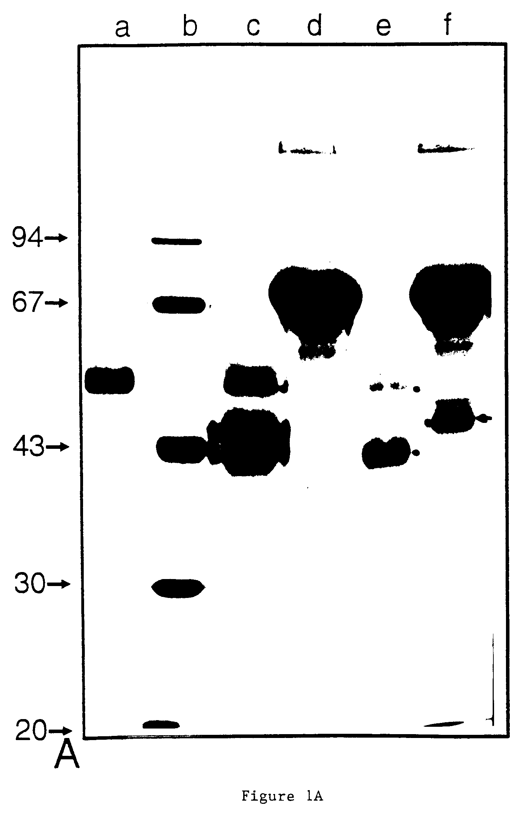

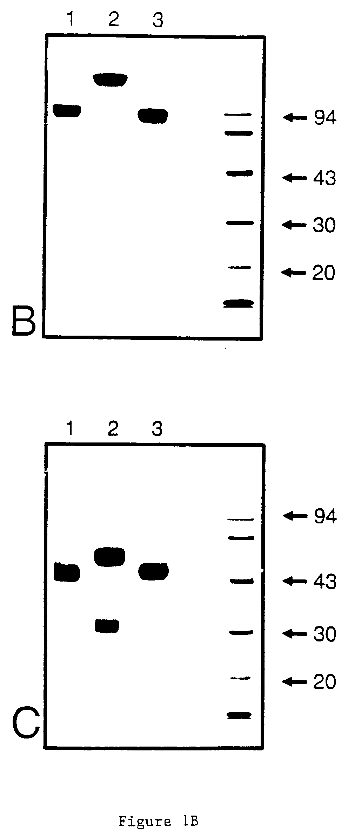

Figure 1 : **Characterisation and purification of camel IgG by affinity chromatography on Protein A and Protein G sepharose (Pharmacia)**

(A) shows, after reduction, the SDS-PAGE protein profile of the adsorbed and non adsorbed fractions of Camelus dromedarius serum. The fraction adsorbed on Protein A and eluted with NaCl 0.15 M acetic acid 0.58% show upon reduction (lane c) three heavy chain components of respectively 50, 46 and 43 Kd and light chain (rabbit IgG in lane a). The fractions adsorbed on a Protein G Sepharose (Pharmacia) derivative which has been engineered to delete the albumin binding region (lane e) and eluted with 0.1 M gly HCl pH 2.7 lacks the 46 Kd heavy chain which is recovered in the non adsorbed fraction (lane f). None of these components are present in the fraction non adsorbed on Protein A (lane d), lane b contains the molecular weight markers. (B) and (C) By differential elution, immunoglobulin fractions containing the 50 and 43 Kd heavy chain can be separated. 5 ml of C. dromadarius serum is adsorbed onto a 5 ml Protein G sepharose column and the column is extensively washed with 20mM phosphate buffer, pH 7.0. Upon elution with pH 3.5 buffer (0.15 M NaCl, 0.58% acetic acid) a 100 Kd component is eluted which upon reduction yields a 43 Kd heavy chain, (lane 1).

After column eluant absorbance has fallen to background level a second immunoglobulin component of 170 Kd can be eluted with pH 2.7 buffer (0.1 M glycine HC). This fraction upon reduction yields a 50 Kd heavy chain and a board light chain band (lane 2).

The fraction non adsorbed on Protein G is then brought on a 5 ml Protein A Sepharose column. After washing and elution with pH 3.5 buffer (0.15 M NaCl, 0.58% acetic acid) a third immunoglobulin of 100 Kd is obtained which consists solely of 46 Kd heavy chains (lane 3).

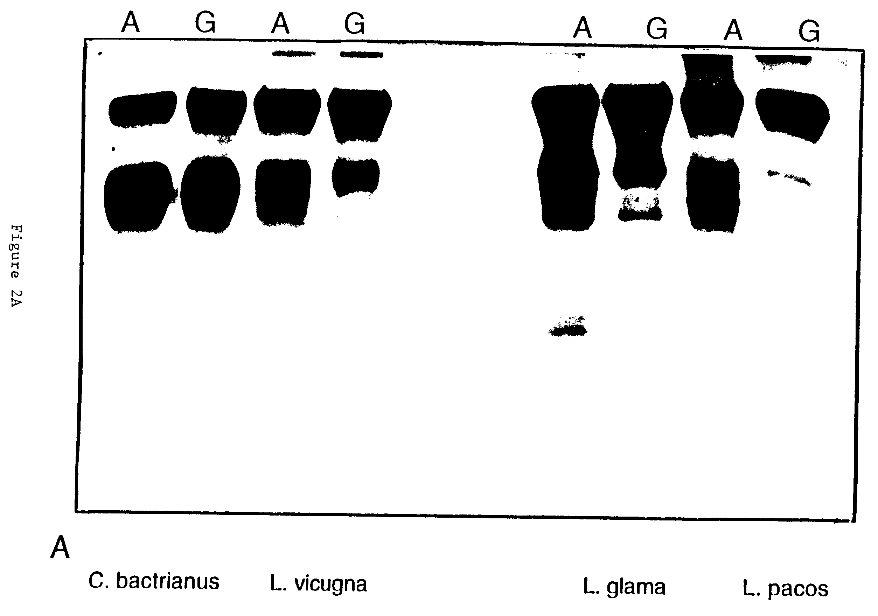

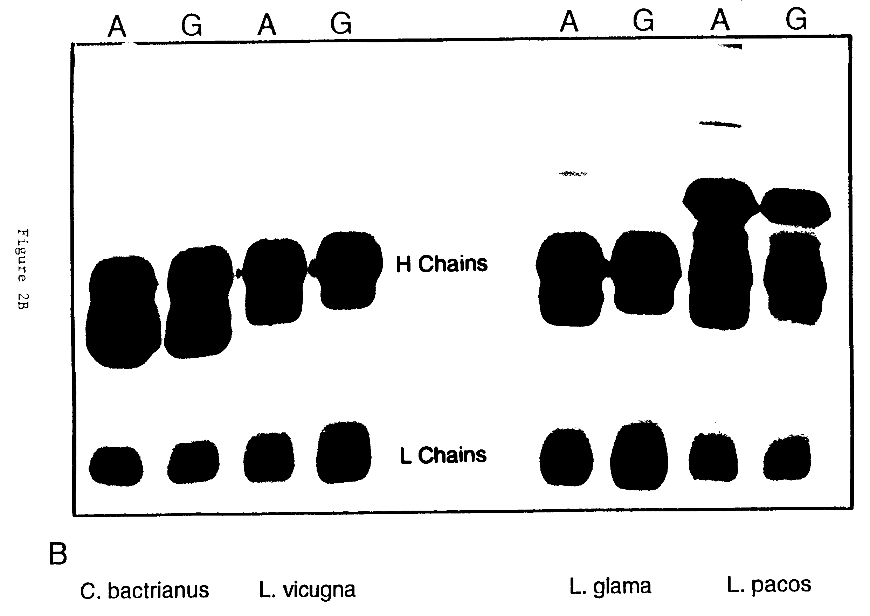

Figure 2 : **Immunoglobulins of Camelus bactrianus, Lama vicugna, Lama glama and Lama pacos to Protein A (A lanes) and to Protein G (G lanes) analyzed on SDS-PAGE before (A) and after reduction (B)**

11

10 $\mu$l of serum obtained from the different species were added to Eppendorf[R] tubes containing 10 mg of Protein A or Protein G sepharose suspended in 400 $\mu$l of pH 8.3 immunoprecipitation buffer (NaCl 0.2. M, Tris 0.01 M; EDTA 0.01 M, Triton X100 1%, ovalbumin 0.1%). The tubes were slowly rotated for 2 hours at 4°C. After centrifugation the pellets were washed 3 times in buffer and once in buffer in which the Triton and ovalbumin had been ommitted. The pellets were then resuspended in the SDS-PAGE sample solution 70 $\mu$l per pellet with or without dithiotreitol as reductant. After boiling for 3 min at 100°C, the tubes were centrifuged and the supernatants analysed.

In all species examined the unreduced fractions (A) contain in addition to molecules of approximately 170 Kd also smaller major components of approximately 100 Kd. In the reduced sample (B) the constituant heavy and light chains are detected. In all species a heavy chain component (marked by an asterisk *) is present in the material eluted from the Protein A but absent in the material eluted from the Protein G.

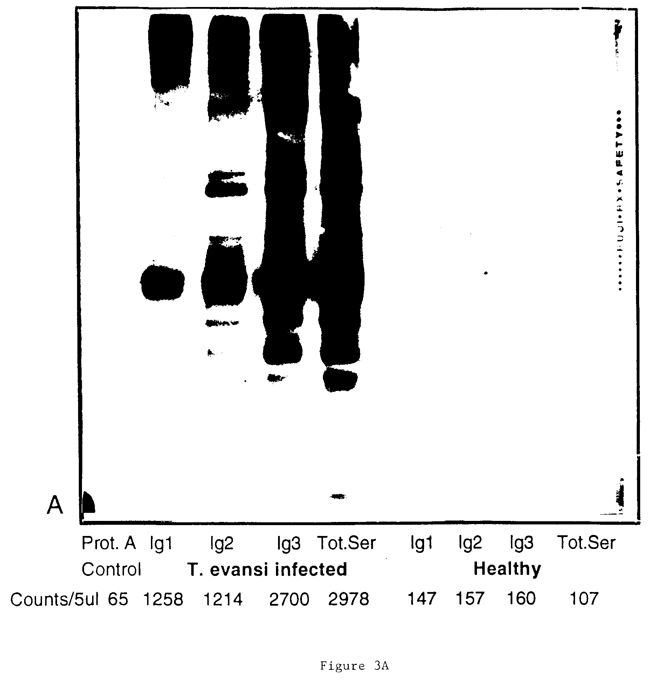

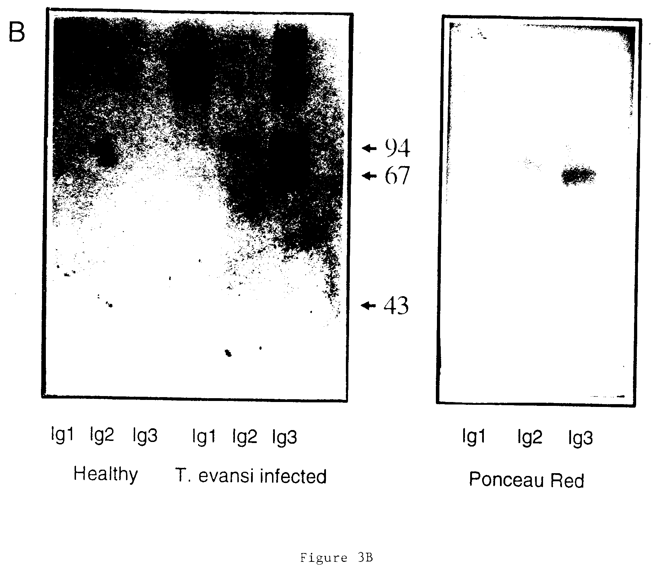

Figure 3 :    **IgG$_1$, IgG$_2$ and IgG$_3$ were prepared from serum obtained from healthy or Trypanosama evansi infected Camelus dromedarius (CATT titer 1/160 (3) and analysed by radioimmunoprecipitation or Western Blotting for anti trypanosome activity**

(A) $^{35}$S methionine labelled Trypanosome evansi antigens lysate (500.000 counts) was added to Eppendorf tubes containing 10 $\mu$l of serum or, 20 $\mu$g of IgG$_1$, IgG$_2$ or IgG$_3$ in 200 $\mu$l of pH 8.3 immunoprecipitation buffer containing 0.1 M TLCK as proteinase inhibitor and slowly rotated at 4°C during one hour. The tubes were then supplemented with 10 mg of Protein A Sepharose suspended in 200 $\mu$l of the same pH 8.3 buffer and incubated at 4°C for an additional hour.

After washing and centrifugation at 15000 rpm for 12 s, each pellet was resuspended in 75 $\mu$l SDS-PAGE sample solution containing DTT and heated for 3 min. at 100°C. After centrifugation in an Eppendorf minifuge at 15000 rpm for 30 s, 5 $\mu$l of the supernatant was saved for radioactivity determination and the reminder analysed by SDS-PAGE and fluorography. The counts/5 $\mu$l sample are inscribed on for each line.

(B) 20 $\mu$g of IgG$_1$, IgG$_2$ and IgG$_3$ from healthy and trypanosome infected animals were separated by SDS-PAGE without prior reduction or heating. The separated samples were then electro transferred to a nitrocellulose membrane, one part of the membrane was stained with Ponceau Red to localise the protein material and the reminder incubated with 1% ovalbumin in TST buffer (Tris 10 mM, NaCl 150 mM, Tween 0.05%) to block protein binding sites.

After blocking, the membrane was extensively washed with TST buffer and incubated for 2 hours with $^{35}$S-labelled trypanosome antigen. After extensive washing, the membrane was dried and analysed by autoradiography. To avoid background and unspecific binding, the labelled trypanosome lysate was filtered through a 45 $\mu$ millipore filter and incubated with healthy camel immunoglobulin and ovalbumin adsorbed on a nitrocellulose membrane.

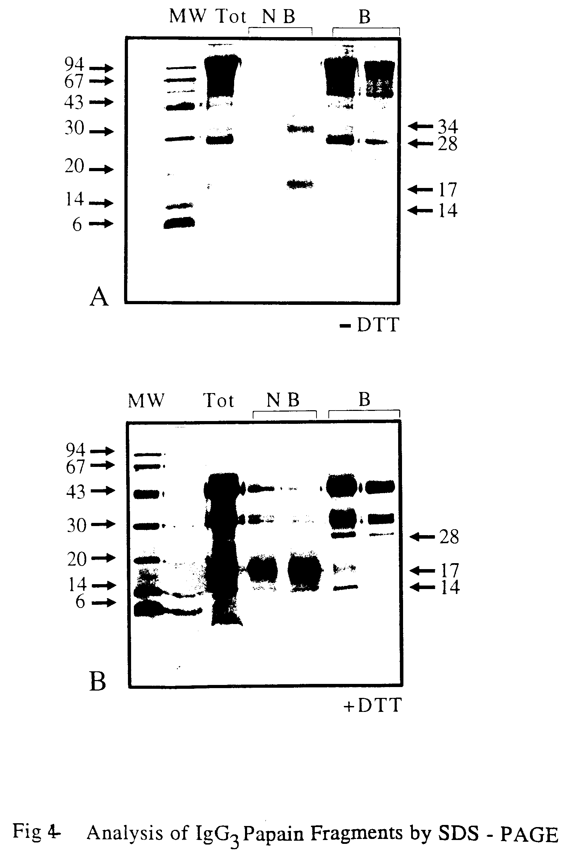

figure 4 :    **Purified IgG3 of the camel, by affinity  chromatography on Protein A Sepharose are partially digested with papain and separated on Protein A sepharose.**

14mg of purified IgG3 were dissolved in 0.1M phosphate buffer ph7.0 containing 2mM EDTA. Yhey were digested by 1 hour incubation at 37°C with mercurypapain (1% enzyme to protein ratio) activated by $5.10^{64}$ M cysteine. The digestion was blocked by the addition ofexcess iodoacetamide $(4.10^{62}M)$(13). After centrifugation of the digest in an ependorf centrifuge for 5min at 15000 rpm, the papain fragments were separated on a protein A Sepharose column into binding (B) and non binding (NB) fractions. The binding fraction was eluted from the column with 0.1M glycine HCl buffer ph 1.7.

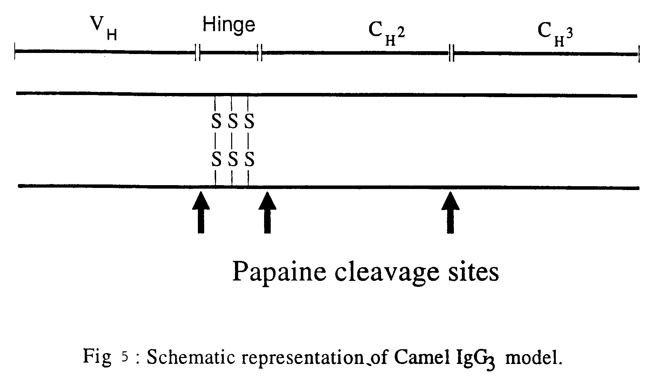

Figure 5 :    **Schematic presentation of a model for IgG3 molecules devoid of light chains.**
Figure 6 :

.    **Schematic representation of immunoglobulins having heavy polypeptide chains and devoid of light chains, regarding conventional four-chain model immunoglobulin**
.    **Representation of a hinge region.**


**I HEAVY CHAIN ANTIBODIES IN CAMELIDS**


When Camelus dromedarius serum is adsorbed on Protein G sepharose, an appreciable amount (25-35%) of immunoglobulins (Ig) remains in solution which can then be recovered by affinity chromatography on Protein A sepharose (fig. 1A). The fraction adsorbed on Protein G can be differentially eluted into a tightly bound fraction (25%) consisting of molecules of an unreduced apparent molecular weight (MW) of 170 Kd and a more weakly bound fraction (30-45%) having an apparent molecular weight of 100 Kd (fig. 1B). The 170 Kd component when reduced yields 50 Kd heavy chains and large 30 Kd light chains. The 100 Kd fraction is totally devoid of light chains and appears to be solely composed of heavy chains which after reduction have on apparent MW of 43 Kd (Fig. 1C). The fraction which does not bind to Protein G can be affinity purified and eluted from a Protein A column as a second 100 Kd component which after reduction appears to be composed solely of 46 Kd heavy chains.

The heavy chain immoglobulins devoid of light chains total up to 75% of the molecules binding to Protein A.

As all three immunoglobulins bind to Protein A we refer to them as IgG : namely IgG$_1$ (light chain and heavy chain $\gamma$1 (50 Kd) binding to Protein G, IgG$_2$ (heavy chain $\gamma$2 (46 Kd) non binding to Protein G and IgG$_3$ (heavy chain $\gamma$3 (43 Kd) binding to Protein G. There is a possibility that these three sub(classes) can be further subdivided.

A comparative study of old world camelids (Camelus bactrianus and Camelus dromedarius) and new world camelids (lama pacos, lama glama, lama vicugna) showed that heavy chain immunoglobulins are found in all species examined, albeit with minor differences in apparent molecular weight and proportion. The new world camelids differs from the old world camelids in having a larger IgG$_3$ molecule (heavy chain immunoglobulin binding to Protein G) in which the constituant heavy chains have an apparent molecular weight of 47 Kd (fig. 2).

The abundance of the heavy chain immunoglobulins in the serum of camelids raises the question of what their role is in the immune response and in particular whether they bear antigen binding specificity and if so how extensive is the repertoire. This question could be answered by examining the immunoglobulins from Trypanosoma evansi infected camels (Camelus dromedarius).

For this purpose, the corresponding fractions of IgG$_1$, IgG$_2$, IgG$_3$ were prepared from the serum of a healthy camel and from the serum of camels with a high antitrypanosome titer, measured by the Card Agglutination Test (3). In radio-immunoprecipitation, IgG$_1$, IgG$_2$ and IgG$_3$ derived from infected camel indicating extensive repertoire heterogeneity and complexity (Fig. 3A) were shown to bind a large number of antigens present in a $^{35}$S methionine labelled trypanosome lysate.

In blotting experiments $^{35}$S methionine labelled trypanosome lysate binds to SDS PAGE separated IgG$_1$, IgG$_2$ and IgG$_3$ obtained from infected animals (Fig. 3B).

This leads us to conclude that the camelid heavy chain IgG$_2$ and IgG$_3$ are bona fide antigen binding antibodies.

An immunological paradigm states that an extensive antibody repertoire is generated by the combination of the light and heavy chain variable V region repertoires (6). The heavy chain immunoglobulins of the camel seem to contradict this paradigm.

Immunoglobulins are characterized by a complex I.E.F. (isoelectric focussing) pattern reflecting their extreme heterogeneity. To determine whether the two heavy chains constituting the IgG$_2$ and IgG$_3$ are identical or not, the isoelectric focussing (I.E.F.) pattern were observed before and after chain separation by reduction and alkylation using iodoacetamide as alkylating agent.

As this alkylating agent does not introduce additional charges in the molecule, the monomers resulting from the reduction and alkylation of a heavy chain homodimer will have practically the same isolectric point as the dimer, whereas if they are derived from a heavy chain heterodimer, the monomers will in most cases differ sufficiently in isoelectric point to generate a different pattern in I.E.F.

Upon reduction, and alkylation by iodoacetamide the observed pattern is not modified for the Camelus dromedarius IgG$_2$ and IgG$_3$ indicating that these molecules are each composed of two identical heavy chains which migrate to the same position as the unreduced molecule they originated from.

In contrast, the I.E.F. pattern of IgG$_1$ is completely modified after reduction as the isoelectric point of each molecule is determined by the combination of the isoelectric points of the light and heavy chains which after separation will each migrate to a different position.

These findings indicate that the heavy chains alone can generate an extensive repertoire and question the contribution of the light chain to the useful antibody repertoire. If this necessity be negated, what other role does the light chain play.

Normally, isolated heavy chain from mammalian immunoglobulins tend to aggregate considerably but are only solubilized by light chains (8, 9) which bind to the C$_H$1 domain of the heavy chain.

In humans and in mice a number of spontaneous or induced myelomas produce a pathological immunoglobulin solely composed of heavy chains (heavy chain disease). These myeloma protein heavy chains carry deletions in the C$_H$1 and V$_H$ domains (10). The reason why full lenght heavy chains do not give rise to secreted heavy chain in such pathological immunoglobulins seems to stem from the fact that the synthesis of Ig involves a chaperoning protein, the immunoglobulin heavy chain binding protein or BIP (11), which normally is replaced by the light chain (12). It is possible that the primordial role of the light chain in the four-chain model immunoglobulins is that of a committed heavy chain chaperon and that the emergence of light chain repertoires has just been an evolutionary bonus.

The camelid $\gamma$2 and $\gamma$3 chains are considerably shorter than the normal mammalian $\gamma$ chain. This would suggest that deletions have occurred in the $C_H$1 domain. Differences in sizes of the $\gamma$2 and $\gamma$3 immunoglobulins of old and new world camelids suggests that deletions occurred in several evolutionary steps especially in the $C_H$1 domain.

## II THE HEAVY CHAIN IMMUNOGLOBULINS OF THE CAMELIDS LACK THE $C_H$1 DOMAIN.

The strategy followed for investigating the heavy chain immunoglobulin primary structure is a combination of protein and cDNA sequencing ; the protein sequencing is necessary to identify sequence streches characteristic of each immunoglobulin. The N-terminal of the immunoglobulin being derived from the heavy chain variable region repertoire only yields information on the $V_H$ subgroups (variable region of the heavy chain) and cannot be used for class or subclass identification. This means that sequence data had to be obtained from internal enzymatic or chemical cleavage sites.

A combination of papaïn digestion and Protein A affinity chromatography allowed the separation of various fragments yielding information on the general structure of IgG3.

The IgG3 of the camel (Camelus dromedarius) purified by affinity chromatography on Protein A Sepharose were partially digested with papaïn and the digest was separated on Protein A Sepharose into binding and non binding fractions. These fractions were analysed by SDS PAGE under reducing and non reducing conditions (fig 4).

The bound fraction contained two components, one of 28 Kd and one of 14.4 Kd, in addition to uncleaved or partially cleaved material. They were well separated by gel electrophoresis ( from preparative 19% SDS-PAGE gels ) under non reducing conditions and were further purified by electroelution( in 50nM amonium bicarbonate, 0.1% (w/v) SDS using a BioRad electroeluter). After lyophilization of these electroeluted fractions, the remaining SDS was eliminated by precipitating the protein by the addition of 90% ethanol, mixing and incubating the mixture overnight at -20°C (14). The precipitated protein was collected in a pellet by centrifuging (15000 rpm, 5min) and was used for protein sequencing.
N-terminal sequencing was performed using the automated Edman chemistry of an Applied Biosystem 477A pulsed liquid protein sequencer. Amino acids were identified as their phenylthiohydantoin (PTH) derivatives using an Applied Biosystem 120 PTH analyser. All chemical and reagents were purchased from Applied Biosystems. Analysis of the chromatographic data was performed using Applied Biosystems software version 1.61. In every case the computer aided sequence analysis was cofirmed by direct inspection of the chromatograms from the PTH analyser. Samples for protein sequencing were dissolved in either 50% (v/v) trifluoroacetic acid(TFA) (28Kd fragment) or 100% TFA ( 14Kd fragment ). Samples of dissolved protein equivalent to 2000 pmol (28Kd fragment) or 500 pmol (14Kd fragment) were applied to TFA-treated glass fibre discs. The glass fibre discs were coated with BioBrene (3mg) and precycled once before use.

N-terminal sequencing of the 28 Kd fragment yields a sequence homologous to the N-terminal part of $\gamma$ $C_H$2domain and hence to the N-terminal end of the Fc fragment. The N-terminal sequence of the 14.4 Kd fragment corresponds to the last lysine of a $\gamma$ $C_H$2 and the N-terminal end of a $\gamma$ $C_H$3 domain (Table 1). The molecular weight (MW) of the papaïn fragments and the identification of their N-terminal sequences led us to conclude that the $C_H$2 and $C_H$3 domains of the $\gamma$3 heavy chains are normal in size and that the deletion must occur either in the $C_H$1 or in the $V_H$ domain to generate the shorted $\gamma$3 chain. The fractions which do not bind to Protein A Sepharose contain two bands of 34 and 17 Kd which are more diffuse is SDS PAGE indicating that they originate from the variable N-terminal part of the molecule (fig 4).

Upon reduction, a single diffuse band of 17 Kd is found indicating that the 34 Kd is a disulfide bonded dimer of the 17 Kd component. The 34 Kd fragment apparently contains the hinge and the N-terminal domain $V_H$.

The protein sequence data can be used to construct degenerate oligonucleotide primers allowing PCR amplification of cDNA or genomic DNA.

It has been shown that the cells from camel spleen imprint cells reacted with rabbit and anti camel immunoglobulin sera and that the spleen was hence a site of synthesis of at least one immunoglobulin class. cDNA was therefore synthetised from camel spleen mRNA. The conditions for the isolation of RNA were the following: total RNA was isolated from the dromedary spleen by the guanidium isothiocyanate method (15). mRNA was purified with oligo T-paramagnetic beads.
cDNA synthesis is obtained using 1$\mu$g mRNA template, an oligodT primer and reverse transcriptase (BOERHINGER MAN). Second strand cDNA is obtained using RNAse H and E coli DNA polymerase I according to the condition given by the supplier.
Relevant sequences were amplified by PCR: 5ng of cDNA was amplified by PCR in a 100$\mu$l reaction

mixture ( 10mM Tris-HCl pH 8.3, 50mM KCl, 15mM $MgCl_2$, 0.01% (w/v) gelatine, 200$\mu$M of each dNTP and 25 pmoles of each primer) overlaid with mineral oil (Sigma).

Degenerate primers containing EcoRI and KpnI sites and further cloned into pUC 18. After a round of denaturing and annealing (94°C for 5 min and 54°C for 5 min),2 units of Taq DNA polymerase were added to the reaction mixture before subjecting it to 35 cycles of amplification:1 min at 94°C (denature) 1min at 54°C (anneal) , 2 min at 72°C (elongate). To amplify DNA sequences between $V_h$ and $C_H2$ domains , (# 72 clones), the PCR was performed in the same conditions with the exception that the annealing temperature was increased to 60°C.

One clone examined (#56/36) had a sequence corresponding to the N-terminal part of a $C_H2$ domain identical to the sequence of the 28 Kd fragment. The availability of this sequence data allowed the construction of an exact 3' primer and the cloning of the region between the N-terminal end of the $V_H$ and the $C_H2$ domain.

5' primers corresponding to the mouse $V_H$ (16) and containing a XhoI restriction site were used in conjunction with the 3' primer in which a KpnI site had been inserted and the amplified sequences were cloned into pBluescript[R]. Clone #56/36 which displayed two internal HaeIII sites was digested with this enzyme to produce a probe to identify PCR positive clones.

After amplification the PCR products were checked on a 1.2% (w/v) agarose gel. Cleaning up of the PCR products included a phenol-chloroform extractio followed by further purification by HPLC ( GEN-PAC FAX column, Waters) and finally by using the MERMAID or GENECLEAN II kit, BIO 101, Inc) as appropriate. After these purification steps, the amplified cDNA was then digested with EcoRI and KpnI for series #56 clones and with XhoI and KpnI for series #72 clones. A final phenol-chloroform extraction preceded the ligation into pUC 18( series #56 clones) or into pBluescript[R] (series #72 clones).

All the clones obtained were smaller that the 860 base pairs to be expected if they possessed a complet $V_H$ and $C_H1$ region. Partial sequence data corresponding to the N-terminal of the $V_H$ region reveals that out of 20 clones, 3 were identical and possibly not independent. The sequences obtained ressemble the human subgroup III and the murine subgroups IIIa and IIIb (Table 2).

Clones corresponding to two different sets of $C_H2$ protein sequences were obtained. A first set of sequences (#72/41) had a N-terminal $C_H2$ region identical to the one obtained by protein sequencing of the 28 Kd papaïn fragments of the $\gamma$3 heavy chain, a short hinge region containing 3 cysteines and a variable region corresponding to the framework (FR4) residues encoded by the J minigenes adjoining the hinge. The $C_H1$ domain is entirely lacking. This cDNA corresponds to the $\gamma$3 chain (Table 3).

In one closely related sequence (#72/1) the proline in position 259 is replaced by threonine.

The sequence corresponding to the $C_H3$ and the remaining part of the $C_H2$ was obtained by PCR of the cDNA using as KpnI primer a poly T in which a KpnI restriction site had been inserted at the 5' end. The total sequence of the $\gamma$3 chain corresponds to a molecular weight (MW) which is in good agreement with the data obtained from SDS PAGE electrophoresis.

The sequence of this $\gamma$3 chain presents similarities with other $\gamma$ chains except that it lacks the $C_H1$ domain, the $V_H$ domain being adjacent to the hinge.

One or all three of the cysteines could be probably responsible for holding the two $\gamma$3 chains together.

These results have allowed us to define a model for the IgG3 molecule based on sequence and papaïn cleavage (fig. 5).

Papaïn can cleave the molecule on each side of the hinge disulfides and also between $C_H2$ and $C_H3$. Under non reducing conditions the $V_H$ domains of IgG3 can be isolated as disulfide linked dimer or as monomer depending on the site of papaïn cleavage.

A second set of clones #72/29 had a slightly different sequence for the $C_H2$ and was characterized by a very long hinge immediately preceded by the variable domain. This hinge region has 3 cysteines at its C-terminal end in a sequence homologeous to the $\gamma$3 hinge. Such second set of clones could represent the IgG2 subclass. For the constant part of the $\gamma$3 and also for the putative $\gamma$2, most clones are identical showing the $\gamma$2 or $\gamma$3 specific sequences. A few clones such as #72/1 however show minor differences. For instance in the case of clones #72/1 two nucleotide differences are detected.

Several $V_H$ regions cDNA's have now been totally or partially sequenced with the exception of a short stretch at the N-terminal end which is primer derived.

Upon translation the majority shows the characteristic heavy chain $Ser_{21}$ $Cys_{22}$ and $Tyr_{90}$ $Tyr_{91}$ $Cys_{92}$ sequences, of the intra $V_H$ region disulfide bridge linking residues 22 and 92. All these clones have a sequence corresponding to the framework 4 (FR4) residues of the variable region immediately preceding the postulated hinge sequence (Table 4). This sequence is generated by the J minigenes and is in the majority of cases similar to the sequence encoded by the human and murine J minigenes. The sequence length between region $Cys_{92}$ and the C-terminal end of the $V_H$ regions is variable and, in the sequences determined, range from 25 to 37 amino-acids as one might expect from the rearrangements of J and D minigenes varying in length.

Several important questions are raised by the sole existence of these heavy chain immunoglobulins in a non pathological situation. First of all, are they bonafide antibodies ? The heavy chain immunoglobulins obtained from trypanosome infected camels react with a large number of parasite antigens as shown in part I of these examples. This implies that the camelid immune system generates an extensive number of binding sites composed of single $V_H$ domains. This is confirmed by the diversity of the $V_H$ regions of the heavy chain immunogobulins obtained by PCR.

The second question is "how are they secreted ?". The secretion of immunoglobulin heavy chains composing four-chain model immunoglobulins does not occur under normal conditions. A chaperoning protein, the heavy chain binding protein, or BIP protein, prevents heavy chains from being secreted. It is only when the light chain dispplaces the BIP protein in the endoplasmatic reticulum that secretion can occur( 13).

The heavy chain dimer found in the serum of human or mice with the so-called "heavy chain disease" lack the $C_H1$ domains thought to harbour the BIP site (14).In the absence of thi domain the BIP protein can no longer bind and prevent the transport of the heavy chains.

The presence in camels of a IgG1 class composed of heavy and light chains making up between 25% and 50% of the total IgG molecules also raises the problem as to how maturation and class switching occurs and what the role of the light chain is. The camelid light chain appears unusually large and heterogeneous when examined in SDS PAGE.

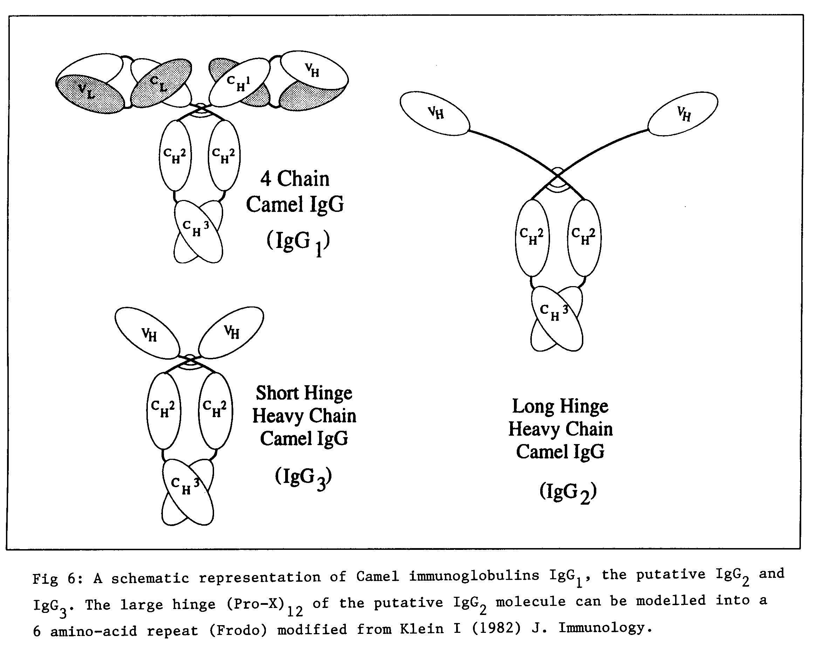

The largest dimension of an isolated domain is 40 Å and the maximum attainable span between binding sites of a conventional IgG with $C_H1$ and $V_H$ will be of the order of 160 Å ($2V_H$ + $2C_H1$)(19). The deletion of $C_H1$ domain in the two types of heavy chain antibodies devoid of light chains, already sequenced has, as a result, a modification of this maximum span (fig. 6). In the IgG3 the extreme distance between the extremities of the $V_H$ regions will be of the order of 80 Å ($2V_H$). This could be a severe limitation for agglutinating or cross linking. In the IgG2 this is compensated by the extremely long stretch of hinge, composed of a 12-fold repeat of the sequence Pro-X (where X is Gln, Lys or Glu) and located N-terminal to the hinge disulfide bridges. In contrast, in the human IgG3, the very long hinge which also apparently arose as the result of sequence duplication does not contribute to increase the distance spanning the two binding sites as this hinge is inter-spersed with disulfide bridges.

The single $V_H$ domain could also probably allow considerably rotational freedom of the binding site versus the Fc domain.

Unlike myeloma heavy chains which result probably from $C_H1$ deletion in a single antibody producing cell, or heavy chain antibodies produced by expression cloning(15); the camelid heavy chain antibodies (devoid of light chains) have emerged in a normal immunological environment and it is expected that they will have undergone the selective refinement in specificity and affinity accompanying B cell maturation.

## TABLE 1

COMPARISON OF THE N-TERMINAL CAMEL OF $C_H2$ and $C_H3$ SEQUENCES WITH THE TRANSLATED cDNA SEQUENCES OF CAMEL IMMUNOGLOBULINS AND WITH THE CORRESPONDING HUMAN γ SEQUENCES

**(Numbering according to Kabat et al, 1987(18)).**

```
                              250                   260          ,           270
CAMEL     γ3 28Kd    - L P G G P S V F V F P P K P K D V L S I S G X P - -
CLONE     #72/1      - L P G G P S V F V F P T K P K D V L S I S G R P - -
CLONE     #72/4      - L P G G P S V F V F P P K P K D V L S I S G R P - -
CLONE     #72/29     - L L G G P S V F I F P P K P K D V L S I S G R P - -
HUMAN     γ1 γ3      - L L G G P S V F L F P P K P K D T L M I S R T P - -
C_H2      γ2        - V A - G P S V F L F P P K P K D T L M I S R T P - -
          γ4         - F L G G P S V F L F P P K P K D T L M I S R T P - -
```

```
                     C_H2 | C_H3
                          |
                     360            370
CAMEL     γ3 14Kd    - K|G Q T R E P Q V Y T L A P X R L E L - -
HUMAN     γ1         - K|G Q P R E P Q V Y T L P P S R D E L - -
CH2/CH3   γ2 γ3      - K|G Q P R E P Q V Y T L P P S R E E M - -
          γ4         - K|G Q P R E P Q V Y T L P P S Q E E M - -
```

EP 0 584 421 A1

```
                    10                   20                   30
Primer Derived | L E S G G G S V Q T G G S L R L S C E I S G L T F D   #DRO 4001

                 L E S G G G S V Q T G G S L R L S C A V S G F S F S   #DRO 3001

                 L E S G G G S E Q G G G S L R L S C A I S G Y T Y G   #DRO 7001

                 L E S G G G S V Q P G G S L T L S C T V S G A T Y S   #DR1 7001

                 L E S G G G S V Q A G G S L R L S C T G S G F P Y G   #DR1 8001
```

EVKL V E S G G G L V E P G G S L R L S C A T S G F T F S   Mouse subgroup III$_A$
                                                          Tepc 15 protein

EVQ L L S G G G L V Q P G G S L R L S C A A S G F T F S   Human subgroup III
                                                          TUR protein

Table 2

A comparison of 4 Fr1 (Framework 1, also designated by FW1) regions of Camel $V_H$ with a human $V_H$III subgroup protein and a mouse $V_H$IIIA subgroup protein.

The invariable subgroup specific residues are in a box.

Human $C_H1$     223                    Hinge                    244          $C_H2$

γ₃ - - K V D K R V  E L K T P L G D T T H T C P R C P ┆

                   E P K S C D T P P P C P R C P ┆

                   E P K S C D T P P P C P R C P ┆- A P E L L G G P S V F L F P -

γ₁ - - K V D K K -  - A E P K S C D K T H T C P P C P ┆- A P E L L G G P S V F L F P -

γ₂ - - K V D K T V  - - - - - - E R K C C V E C P P C P ┆- A P P V A G - P S V F L F P -

γ₄ - - K V D K R V  - - - - - - E S K Y G P P C P S C P ┆- A P E F L G G P S V F L F P -

Camel $V_H$                          Hinge                                $C_H2$

#72/4 - V T V S S  - - - - - - G T N E V C K C P K C P ┆- A P E L P G G P S V F V F P -

                   K P E P C T C P K C P ┆- A P E L L G G P S V F I F P -

                   Q P Q P K P Q P

#72/29 - V T V S S  - - - E P K I P Q P Q P K P Q P Q P ┆

Table 3   Comparison of clone 72/4 and clone 72/29 hinge regions with the
          human γ hinges and the adjacent sequences.

EP 0 584 421 A1

| | J Genes | Framework 4 |
|---|---|---|
| **Human** | J1, J4, J5 | W G Q G T L V T V S S |
| | J2 | W G R G T L V T V S S |
| | J6 | W G Q G T T V T V S S |
| | J3 | W G Q G T M V T V S S |
| **Murine** | J1 | W G Q G T T L T V S S |
| | J2 | W G Q G T L V T V S S |
| | J3 | W G Q G T S V T V S A |
| | J4 | W G A G T T V T V S S |
| **Camel** | cDNA Clones | |
| | Clones | W G Q G T Q V T V S S |
| | 1 Clone | W G Q G T L V T V S S |

**Table 4**

Comparison of the Framework residues found in the Camel $V_H$ region with the Framework 4 residues corresponding to the consensus region of the Human and Mouse J minigenes.

**REFERENCES**

1. Ward, E.S., Güssow, D., Griffits, A.D., Jones, P.T. and Winter G. Nature 341, 544-546 (1989).
2. Ungar-Waron H., Eliase E., Gluckman A. and Trainin Z. Isr. J. Vet. Med., 43, 198-203 (1987).
3. Bajyana Songa E. and Hamers R., Ann. Soc. Belge Méd. trop., 68, 233-240 (1988).
4. Edelman G.M., Olins D.E., Gally J.A. and Zinder N.D., Proc. Nat. Acad. Sc., 50, 753 (1963).
5. Franek F. and Nezlin R.S., Biokhimiya, 28, 193 (1963).
6. Roitt I.M., Brostof J. and Male D.K., Immunology, Gower Med. Pub. London. New-York, p.9.2. (1985).
7. Schiffer M., Girling R.L., Ely K.R. and Edmundson B., Biochemistry, 12, 4620-4631 (1973).
8. Fleischman J.B., Pain R.H. and Porter R.R., Arch. Biochem. Biophys, Suppl. 1, 174 (1962).

9. Roholt O., Onoue K. and Pressman D., PNAS 51, 173-178 (1964).

10. Seligmann M., Mihaesco E., Preud'homme J.L., Danon F. and Brouet J.C., Immunological Rev., 48, 145-167 (1979).

11. Henderschot L., Bole D., Köhler G. and Kearney J.F., The Journal of Cell Biology, 104, 761-767 (1987).

12. Henderschot L.M., The Journal of Cell Biology, 111, 829-837 (1990).

13. Hamers-Casterman, C., E. Wittouck, W. Van der Loo and R. Hamers, Journal of Immunogenetics, 6, 373-381 (1979).

14. Applied Biosystems - Ethanol Precipitation of Electro Eluted Electrodialysed Sample. Issue n° 27.

15. Maniatis, T. E.F. Fritsch and J. Sambrook, Molecular Cloning. A Laboratory Manual (1988).

16. Sastry et al., PNAS, 86, 5728, (1989).

17. Sanger, F., S. Nicklen and A.R. Coulson, Proc. Natl. Acad. Sci., U.S.A., 74, 5463-5467 (1977).

18. Kabat E.A., Tai Te Wu, M. Reid-Miller, H.M. Perry and K.S. Gottesman, U.S. Dpt of Health and Human Services, Public Health Service, National Institutes of Health (1987).

19. Valentine, R.C. and N.M. Geen, J.M.B., 27, 615-617 (1967).

## Claims

1. Isolated immunoglobulin, characterized in that it comprises two heavy polypeptide chains sufficient for the formation of a complete antigen binding site or several antigen binding sites, this immunoglobulin being further devoid of light polypeptide chains.

2. Immunoglobulin according to claim 1, characterized in that the amino acid sequence of its variable region contains in position 45 an amino acid which is different from a leucine, or proline or glutamine residue.

3. Immunoglobulin according to anyone of claims 1 to 2, characterized in that its heavy polypeptide chains are devoid of a so-called first domain in their constant region ($C_H1$).

4. Immunoglobulin according to claim 1 to 3, characterized in that it comprises an antigen binding site or several antigen binding sites.

5. Immunoglobulin according to claim 1 to 3, characterized in that each variable region of each heavy chain contains at least one antigen binding site.

6. Immunoglobulin according to anyone of claims 1 to 5, characterized in that it is a type G immunoglobulin of class 2 (IgG2).

7. Immunoglobulin according to anyone of claims 1 to 5, characterized in that it is a type G immunoglobulin of class 3 (IgG3).

8. Immunoglobulin according to anyone of claims 1 to 7, characterized in that it is a Camelid immunoglobulin.

9. Immunoglobulin according to anyone of claims 1 to 6 obtainable by purification from the serum of Camelids, characterized in that :
   - it is not adsorbed by chromatography on Protein G Sepharose column,
   - it is adsorbed by chromatography on Protein A Sepharose column,
   - it has a molecular weight of around 100 Kd after elution with a pH 4.5 buffer (0.15 M NaCl, 0.58% acetic acid adjusted to pH 4.5 by NaOH),
   - it consists of heavy $\gamma$2 polypeptide chains of a molecular weight of around 45 Kd preferably 46 Kd after reduction.

10. Immunoglobulin according to anyone of claims 1 to 5 or 7, obtainable by purification from the serum of Camelids, characterized in that :
    - it is adsorbed by chromatography on a Protein A Sepharose column,
    - it has a molecular weight of around 100 Kd after elution with a 3.5 buffer (0.15 M NaCl, 0.58% acetic acid),

- it is adsorbed by chromatography on a Protein G Sepharose column and eluted with pH 3.5 buffer (0.15 M NaCl, 0.58% acetic acid).
- it consists of heavy $\gamma3$ polypeptide chains of a molecular weight of around 45 Kd in particular between 43 and 47 Kd after reduction.

11. Immunoglobulin according to anyone of claims 1 to 7, characterized in that it comprises 4 frameworks in its variable region, which frameworks comprise an amino-acid sequence selected from the following sequences :
for the framework 1 domain

```
G G S V Q T G G S L R L S C E I S G L T F D

G G S V Q T G G S L R L S C A V S G F S F S

G G S E Q G G G S L R L S C A I S G Y T Y G

G G S V Q P G G S L T L S C T V S G A T Y S

G G S V Q A G G S L R L S C T G S G F P Y S

G G S V Q A G G S L R L S C V A G F G T S


G G S V Q A G G S L R L S C V S F S P S S
```

for the framework 4 domain

```
W G Q G T Q V T V S S

W G Q G T L V T V S S

W G Q G A Q V T V S S

W G Q G T Q V T A S S

R G Q G T Q V T V S L
```

12. Immunoglobulin according to anyone of claims 1 to 6 or 8, characterized in that its constant region comprises $C_H2$ and $C_H3$ domains comprising an amino-acid sequence selected from the following sequences :
for the $C_H2$ domain:

```
APELLGGPTVFIFPPKPKDVLSITLTP

APELPGGPSVFVFPTKPKDVLSISGRP

APELPGGPSVFVFPPKPKDVLSISGRP

APELLGGPSVFIFPPKPKDVLSISGRP
```

for the $C_H3$ domain:
GQTREPQVYTLA

13. Immunoglobulin according to anyone of claims 1 to 9, characterized in that its hinge region comprises from 0 to 50 amino-acids.

14. Immunoglobulin according to claim 13, characterized in that its hinge region comprises an amino-acid sequence selected from the following sequences :
GTNEVCKCPKCP

22

or,

EPKIPQPQPKPQPQPQPQPKPQPKPEPECTCPKCP

**15.** Immunoglobulin according to anyone of claims 1 to 14, characterized in that all or a part of its constant region is replaced by all or part of the constant region of a human antibody.

**16.** Fragment of an immunoglobulin according to anyone of claims 1 to 15, characterized in that it is selected from the following group :
- a fragment corresponding to one heavy polypeptide chain of an immunoglobulin devoid of light chains,
- fragments obtained by enzymatic digestion of the immunoglobulins of the invention, especially those obtained by partial digestion with papaïn leading to the Fc fragment (constant fragment) and leading to $Fv_Hh$ fragment (containing the antigen binding sites of the heavy chains) or its dimer $F(V_Hh)_2$, or a fragment obtained by further digestion with papaïn of the Fc fragment, leading to the Fc' fragment corresponding to the C-terminal part of the Fc fragment,
- homologous fragments obtained with other proteolytic enzymes,
- a fragment of at least 10 preferably 20 amino acids of the variable region of the immunoglobulin, or the complete variable region, especially a fragment corresponding to the isolated $V_H$ domains or to the $V_H$ dimers linked to the hinge disulphide,
- a fragment corresponding to the hinge region of the immunoglobulin,or to at least 6 amino acids of this hinge region,
- a fragment of the hinge region comprising a repeated sequence of Pro-X,
- a fragment corresponding to at least 10 preferably 20 amino acids of the constant region or to the complete constant region of the immunoglobulin.

**17.** An amino-acid sequence characterized in that it comprises a repeated sequence Pro-X, X being any amino-acid and preferably Gln, Lys or Glu, the sequence containing advantageously at least 3 repeats of Pro-X and especially a fragment composed of a 12-fold repeat of the sequence Pro-X.

**18.** Nucleotide sequence, characterized in that it codes for all or part of a protein which amino-acid sequence comprises a peptide sequence selected from the following :

**VTVSSGTNEVCKCPKCPAPELPGGPSVVFVVFP,**

**VTVSSEPKIPQPQPKPQPQPQPQPKPQPKPEPECTCPKCPAPELLGGPSVFIFP**

**GTNEVCKCPKCP**

**APELPGGPSVFVFP**

**EPKIPQPQPKPQPQPQPQPKPQPKPEPECTCPKCP**

**APELLGGPSVFIFP**

**19.** Process for the preparation of a monoclonal antibody directed against a determined antigen, the antigen binding site of the antibody consisting of heavy polypeptide chains and which antibody is further devoid of light polypeptide chains, which process comprises :
- immortalizing lymphocytes, obtained for example from the peripheral blood of Camelids previously immunized with a determined antigen, with myeloma cells, in order to form a hybridoma,
- culturing the immortalized cells formed and recovering the cells producing the antibodies having the desired specificity.

**20.** Process for the preparation of antibodies directed against determined antigens, comprising the steps of :
- cloning into vectors, especially into phages and more particularly filamentous bacteriophages, DNA or cDNA sequence obtained from lymphocytes of Camelids previously immunized with determined antigens,
- transforming prokariotic cells with the above vectors in conditions allowing the production of the antibodies,

23

- selecting the appropriate antibody by subjecting the transforming cells to antigen-affinity selection,
- recovering the antibodies having the desired specificity.

21. Process according to claim 20 wherein the cloning vector is a plasmid or a eukaryotic virus and the transformed cell is a eukaryotic cell, especially a yeast cell, mammalian cell, plant cell or protozoair cell.

22. Process according to claim 20 wherein the cloning vector is a plasmid capable of expressing the immunoglobulin in the bacterial membrane.

23. Process according to claim 20, wherein the cloning vector is a plasmid capable of expressing the immunoglobulin as a secreted protein.

24. Immunoglobulin according to anyone of claims 1 to 18, characterized in that it is directed against an antigen such as one of a bacteria, a virus, a parasite, or against a protein, hapten, carbohydrate or nucleic acid.

25. Immunoglobulin according to anyone of claims 1 to 18 characterized in that it is directed against an immunoglobulin idiotype.

26. Immunoglobulin according to anyone of claims 1 to 18 characterized in that it is directed against a cellular receptor or membrane protein.

27. Immunoglobulin according to anyone of claims 1 to 18, characterized in that it has a catalytic activity.

28. Immunoglobulin according to anyone of claims 1 to 18, or a fragment according to claim 16, characterized in that it is conjugated with a toxin.

29. Use for a fragment according to claim 17 for coupling protein domains or a protein and a ligand.

30. Use of the hinge region or of a fragment of the hinge region of an immunoglobulin according to anyone of claims 1 to 16, for coupling protein domains or a protein and a ligand.

31. Immunoglobulin according to anyone of claims 1 to 16, characterized in that it is a heterospecific antibody.

32. Recombinant vector characterized in that it comprises a nucleotide sequence according to claim 19 and in that it is a plasmid, a phage especially a bacteriophage, a virus, a YAC, a cosmid.

33. Recombinant cell or organism characterized in that it is modified by a vector according to claim 32.

34. A cDNA library composed of nucleotide sequences coding for a heavy-chain immunoglobulin according to claim 1 or 2, such as obtained by performing the following steps:
    a) treating a sample containing lymphoïd cells, especially periferal lymphocytes, spleen cells, lymph nodes or another lyphoïd tissue from a healthy animal, especially selected among the Camelids, in order to separated the B-lymphocytes,
    b) separating polyadenylated RNA from the other nucleic acids and components of the cells,
    c) reacting the obtained RNA with a reverse transcriptase in order to obtain the corresponding cDNA,
    d) contacting the amplified DNA of step e) with 5' primers corresponding to mouse $V_H$ domain of four-chain immunoglobulins, which primer contains a determined restriction site, for example an XhoI site and with 3' primers corresponding to the N-terminal part of a $C_H2$ domain,
    e) amplifying the DNA,
    f) cloning the amplified sequence in a vector, especially in a bluescript vector,
    g) recovering the clones hybridizing with a probe corresponding to the sequence coding for a constant domain from an isolated heavy-chain immunoglobulin.

35. A modified 4-chain immunoglobulin or a fragment thereof, the $V_H$ regions of which has been partialy replaced by specific sequences or amino acids of heavy chain immunoglobulins according to anyone of claims 1 to 16.

36. A modified 4-chain immunoglobulin or a fragment thereof, according to claim 35, wherein the leucine, proline or glutamine in position 45 of the $V_H$ regions has been replaced by other amino acids and preferably by arginine, glutamic acid or cysteine.

37. A modified 4-chain immunoglobulin or a fragment thereof, in which the CDR loops of the region are linked to other parts of the V region by the introduction of paired cysteines, in particular in which the $CDR_3$ loop is linked to the $FW_2$ or $CDR_1$ and more especially where the cysteine of the $CDR_3$ of the $V_H$ is linked to a cysteine in position 31 or 33 of $FW_2$ or in position 45 of $CDR_2$.

Figure 1A

Figure 1B

Figure 2A

Figure 2B

|  | Prot. A | Ig1 | Ig2 | Ig3 | Tot.Ser | Ig1 | Ig2 | Ig3 | Tot.Ser |
|---|---|---|---|---|---|---|---|---|---|
|  | Control | | **T. evansi infected** | | | | **Healthy** | | |
| Counts/5ul | 65 | 1258 | 1214 | 2700 | 2978 | 147 | 157 | 160 | 107 |

Figure 3A

B

←94
←67

←43

Ig1  Ig2  Ig3      Ig1  Ig2  Ig3

Healthy      T. evansi infected

Ig1      Ig2      Ig3

Ponceau Red

Figure 3B

Fig 4   Analysis of IgG$_3$ Papain Fragments by SDS - PAGE

$V_H$     Hinge     $C_H 2$     $C_H 3$

S S S

S S S

Papaine cleavage sites

Fig 5 : Schematic representation of Camel $IgG_3$ model.

Fig 6: A schematic representation of Camel immunoglobulins $IgG_1$, the putative $IgG_2$ and $IgG_3$. The large hinge $(Pro-X)_{12}$ of the putative $IgG_2$ molecule can be modelled into a 6 amino-acid repeat (Frodo) modified from Klein I (1982) J. Immunology.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | PLANT MOLECULAR BIOLOGY vol. 16, no. 4, April 1991, DORDRECHT NL pages 663 - 670 RAINES ET AL. 'A novel proline-rich protein from wheat' * figure 1 * | 17,29 | C12N15/13 C07K15/06 C12P21/08 C12N5/10 A61K39/395 |
| A,D | ISRAEL JOURNAL OF  VETERINARY MEDECINE vol. 43, no. 3, March 1987, pages 198 - 203 UNGAR -WARON ET AL. 'Dromedary IgG:purification, characterization and quantitation in sera of dams and newborns' * page 202, column 2, paragraph 3; figure 2 * | 1-37 | |
| A,D | NATURE. vol. 341, no. 6242, 12 October 1989, LONDON GB pages 544 - 546 WARD ET AL. 'Binding activities of a repertoire of single immunoglobulin variable domains secreted from Escherichia coli' * the whole document * | 1-37 | |

TECHNICAL FIELDS SEARCHED (Int. Cl.5 )

C12N
C12P
C07K
A61K

The present search report has been drawn up for all claims

| Place of search | Date of completion of the search | Examiner |
|---|---|---|
| THE HAGUE | 28 APRIL 1993 | CUPIDO M. |

EPO FORM 1503 03.82 (P0401)